# EUROPEAN PATENT APPLICATION

(11) **EP 0 683 974 A1**
(43) Date of publication of application: **29.11.1995**
(21) Application number: 95201027.0
(22) Date of filing: 21.04.1995
(51) Int. Cl.: A01J 5/017, A61B 5/103

(54) **Method for checking the condition of human or animal skin**

(30) Priority: 25.04.1994 NL 9400658
(71) Applicant: STICHTING INSTITUUT VOOR MECHANISATIE ARBEID EN GEBOUWEN, NL-6700 AA Wageningen (NL)
(72) Inventor: van Heulen, Simon Franciscus, NL-3912 AA Rhenen (NL); Hogewerf, Pieter Hendrik, NL-6932 GH Westervoort (NL); Kornet, Johannes George, NL-6708 BW Wageningen (NL)
(74) Representative: de Bruijn, Leendert C.

(57) **Abstract**

Method for checking the skin surface of a human or an animal for contamination, such as checking the teat of a cow's udder for contamination by manure, wherein one or more sources of electromagnetic radiation are directed onto the skin surface to be tested and transmit electromagnetic oscillation towards said surface, whilst one or more receivers for electromagnetic radiation are likewise directed onto the said skin surface, for receiving electromagnetic radiation originating from the said skin surface, and wherein measurement is carried out in the electromagnetic radiation range in which a clear difference is found between the radiation intensity reflected by a skin surface without contamination or having a moist contamination and that having an essentially dry contamination.

## Description

The invention relates to a method for checking the skin surface of a human or an animal, such as checking a teat of a cow for contamination by manure, wherein one or more sources of electromagnetic radiation are directed onto the skin surface to be inspected and transmit electromagnetic vibration towards said surface, whilst one or more receivers for electromagnetic radiation are likewise directed onto the said skin surface, for receiving electromagnetic radiation originating from the said skin surface, and wherein measurement is carried out in the electromagnetic radiation range in which a clear difference is found in the radiation intensity reflected by a skin surface having a deviating condition caused by differences in moisture content.

A method for detecting contamination on the skin of the teat of a cow is disclosed, for example, in W092/19098, with which method a reflection pattern of electromagnetic radiation is determined. This publication further provides neither clear references to the frequency levels used nor any insight into the determination of differences in reflection patterns on the basis of a deviation in the moistness of the inspected surface.

The aim of the invention is to provide a practical method for determining the condition or the hygiene of the skin surface, for example the teat of a cow, but could equally well be applied for, for example, checking the condition, for example, the cleanliness, of human hands. By means of the invention, use is made of the newly provided insight that the nature of the moisture on a surface has an appreciable effect on the reflection pattern of electromagnetic radiation, using which insight it is possible to determine the presence of dried-on material on a skin surface. Consequently, the aim is therefore not, as is customary, to determine the presence of a substance (such as, for example, "chlorophyll") or a colour by means of the measured reflection pattern.

This insight according to the invention can be used, for example, in order to establish the presence of, for example, manure on a teat of a cow. The manure usually has a drier surface than an adjacent clean area of skin. Only the surface of manure which has only just come onto the skin is too moist for a distinction to be made.

The "fresh" manure can be removed simply, for example completely automatically with the aid of the cleansing water jets now customary in automatic milking. Moreover, observations have shown that manure dries very rapidly on the teat skin, as a result of which the percentage of animals having wet manure on the teat is very low. However, the water jets have been found to be insufficiently capable of removing, from a teat, the manure which has already been on the teat for a somewhat longer period, or other contamination. By now checking the skin surface for the presence of such contaminants before the treatment with water jets or another cleaning treatment, an adequate and effective supplementary treatment can be carried out for the removal thereof.

It has been found that the effect of the invention is clearest in the case of measurements above 1200 nm. At present, preference is given to measurements in the wavelength range of 1200 to 1600, preferably at 1300 or 1550 nm. However, measurements at higher wavelength levels are also used. The wavelength range indicated above is practical - since standard components for this are currently commercially available. Application of the invention is not restricted to inspection for contamination. It is anticipated that other deviations in conditions which give rise to a difference in the moisture content of the surface of the skin, such as callosity, can be determined by means of the invention.

The invention is explained in more detail below with reference to various reflection patterns, shown in the appended drawing, of the living skin of various teat types in cows, with and without contamination by manure. In the figures:
Fig. 1 shows the reflection pattern of a clean black teat;
Fig. 2 shows the reflection pattern of a clean variegated teat, measured on the black-coloured part of the teat;
Fig. 3 shows the reflection pattern of a clean white teat;
Fig. 4 shows the reflection pattern of a clean variegated teat measured on the white-coloured part of the teat;
Fig. 5 shows the reflection pattern of dry manure, which has adhered to the skin of each of the teat types indicated above;
Figs. 6 and 7 show the reflection pattern of manure present on each type (see Figs. 1-4) of teat, during the drying process of the manure;
Fig. 8 shows the reflection pattern of manure for various moisture contents, measured at a wavelength of 1300 nm; and
Fig. 9 shows the reflection pattern of manure for various moisture contents, measured at a wavelength of 1550 nm. The reflection is in each case expressed in the dimensionless unit "p".

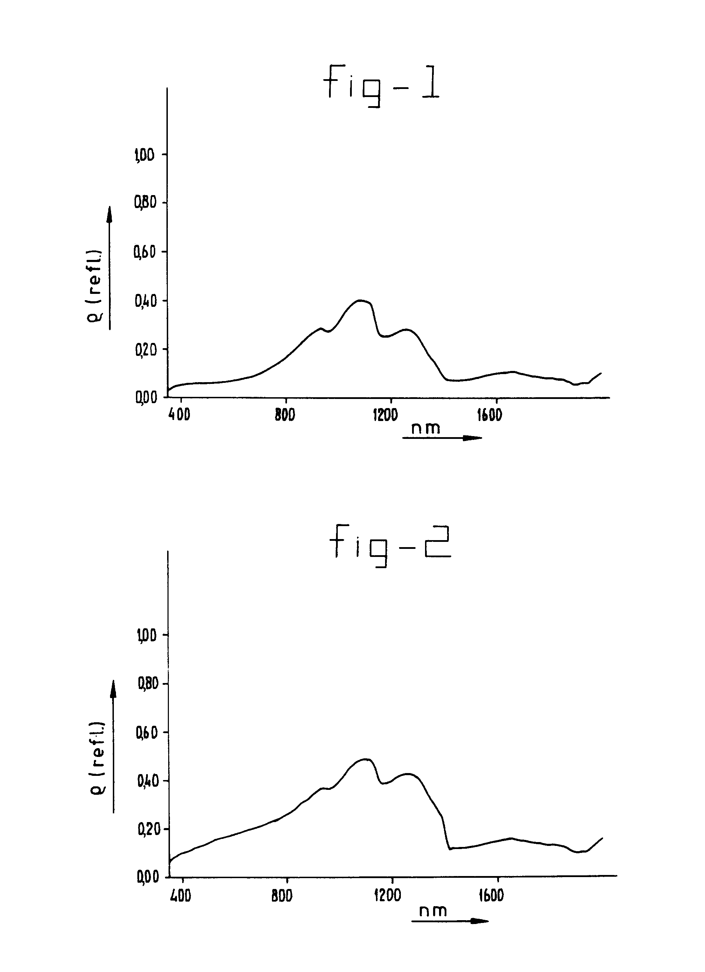

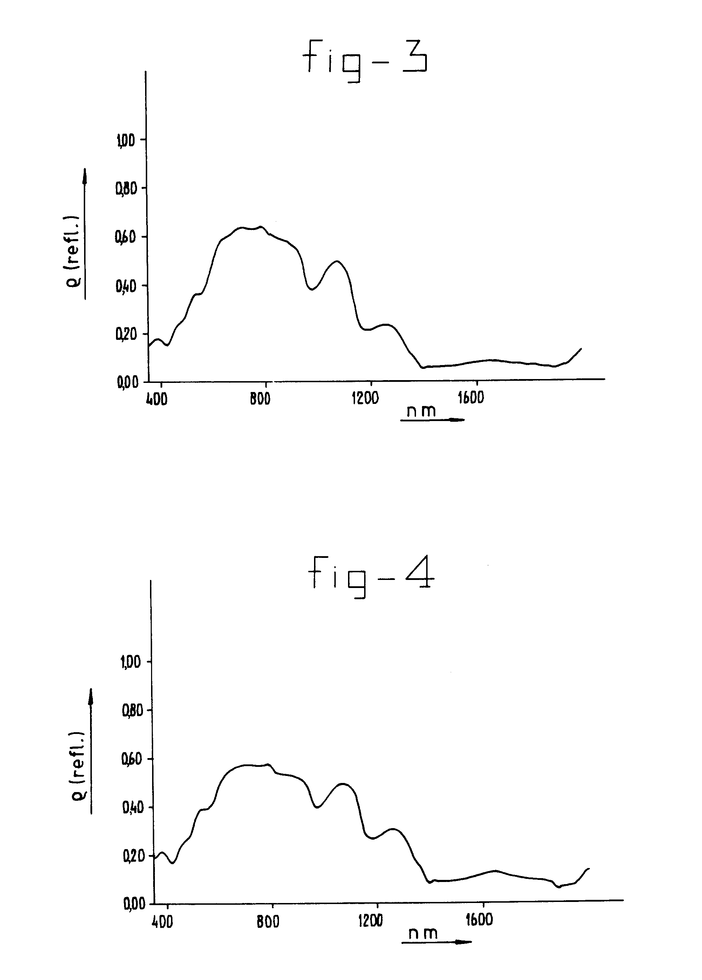

Comparison of Figures 1 to 5 shows that the reflection patterns (of the various teat types: black, variegated, white) show no appreciable differences between them. In each case a clear reduction in the reflection can be seen in the region from 1200 to 1600 nm, after which the reflection level remains at a virtually constant, relatively low level from about 1300 nm.

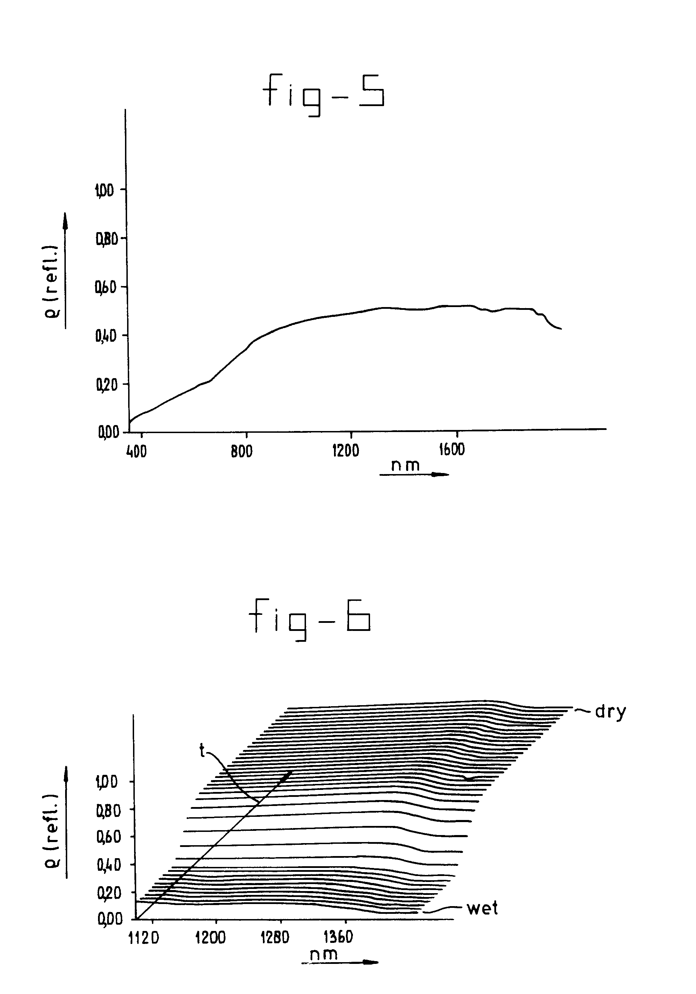

Fig. 5, which relates to essentially dry manure on the skin, shows a gradual increase in the reflection up to 800 nm, after which the reflection level remains at a relatively high, essentially constant level to beyond 1600 nm. Comparison of Figures 1-4 with Fig. 5 shows that from about 900 nm the reflection level from clean teats is always below the reflection level from dry manure. From about 1300 nm, the difference in level is appreciable. Thus, in the case of measurements at a wavelength above about 1300 nm there will be a very clear distinction between a clean skin area and a contaminated skin area.

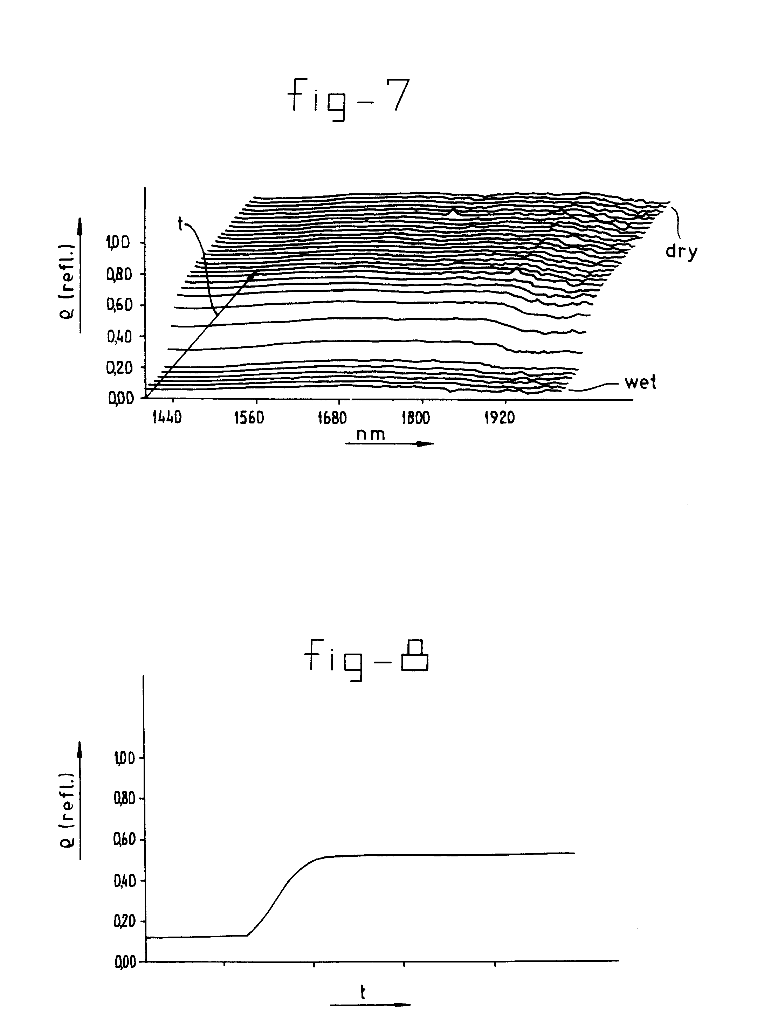

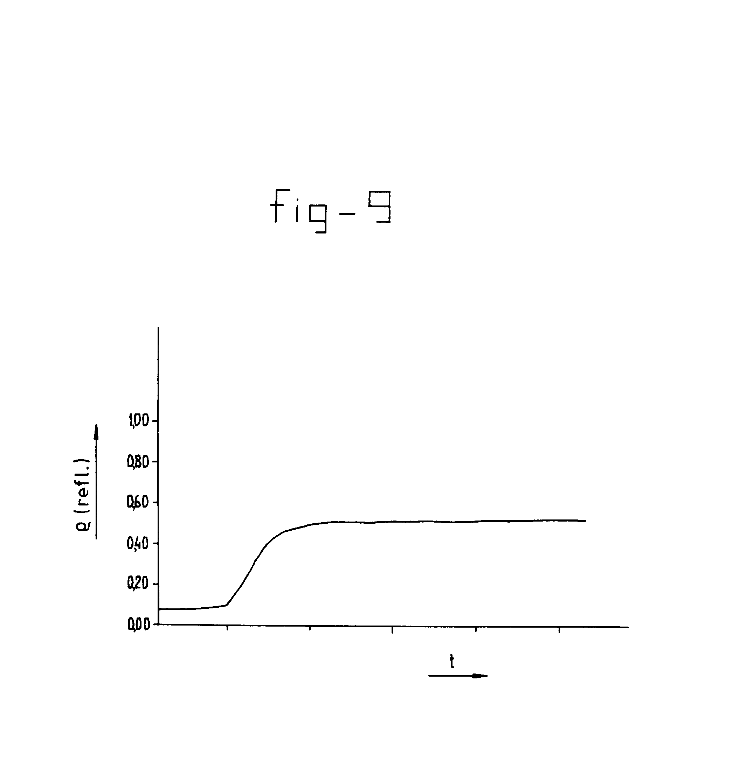

Consideration of Figures 6 to 9 shows that manure which has just been applied, that is to say still with a fairly moist surface (moisture content probably 70-90 %), yields a relatively low reflection level which hardly differs from one figure to the other. Manure which has been present on the skin for somewhat longer, with a surface which has already started to dry on (moisture content probably 0-40 %), shows an appreciably higher reflection level.

It has been found that the above results can be applied not only to contamination with manure but also to other types of contamination having the same proportion of moisture. For example, the presence of blood can also be detected using the method according to the invention. Fresh blood can be removed very easily by means of the conventional water jets. If clotted blood is detected, an alarm signal can, for example, be given immediately, so that, in respect of cows or other animals, the person caring for the animals is automatically informed that an injury has occurred. The method according to the invention can be carried out, for example, using a device such as is shown in Figs 1, 2 and 3 of W092/19098 and the associated description, the various features being adapted to operate in the wavelength ranges indicated in the present description.

## Claims

1. Method for checking the skin surface of a human or an animal, such as checking a teat of a cow for contamination by manure, wherein one or more sources of electromagnetic radiation are directed onto the skin surface to be inspected and transmit electromagnetic vibration towards said surface, whilst one or more receivers for electromagnetic radiation are likewise directed onto the said skin surface, for receiving electromagnetic radiation originating from the said skin surface, and wherein measurement is carried out in the electromagnetic radiation range in which a clear difference is found in the radiation intensity reflected by a skin surface having a deviating condition caused by differences in moisture content.

2. Method according to Claim 1, wherein said method is operated in the wavelength range of electromagnetic radiation from 400 to 3000, in particular from 400 to 2000 and more particularly from 900 to 2000 nm, that is to say essentially in the wavelength range of visible light and/or infra-red light.

3. Method according to Claim 2, wherein the measurements are carried out between 1200 and 1600 nm, in particular at 1300 and 1550 nm.
